# EUROPEAN PATENT APPLICATION

(11) **EP 2 224 235 A1**
(43) Date of publication of application: **01.09.2010**
(21) Application number: 08853570.3
(22) Date of filing: 28.11.2008
(51) Int. Cl.: G01N 27/62, G01N 33/576

(54) **METHOD OF DIAGNOSTIC HEPATIC DISEASE BY SUGAR CHAIN ANALYSIS**

(30) Priority: 30.11.2007 JP 2007310285
(71) Applicant: National University Corporation Hokkaido University, Sapporo-shi Hokkaido 060-0808 (JP); Shionogi&Co., Ltd., Osaka-shi, Osaka 5410045 (JP)
(72) Inventor: NISHIMURA, Shin-ichiro, Kita 21-jyo Noshi 11-chome, Kita-ku Sapporo-shi, Hokkaido 001-0021 (JP); TODO, Satoru, Sapporo-shi Hokkairo 060-8638 (JP)
(74) Representative: White, Nina Louise
(86) International application number: PCT/JP2008/071709
(87) International publication number: WO 2009/069776

(57) **Abstract**

It is intended to provide a novel diagnosis method for a hepatic disease, in particular, HCC. A serum sugar chain usable as a novel biomarker is provided by analyzing the relationship between the quantitative expression profiles of sugar chains in the serum and individual disease conditions and thus finding a sugar chain showing a change with the progression of a hepatic disease.

## Description

### TECHNICAL FIELD

The present invention relates to a method for diagnosing a hepatic disease using a sugar chain analysis.

### BACKGROUND ART

Hepatocellular carcinoma (HCC) is one of the most common cancers on a global scale and it causes the deaths of one million people each year. Many of HCC develop from viral hepatitis. HCC derived from hepatitis C virus (HCV) is the most common HCC and the percentage of the total is 70-80 %. HCC derived from hepatitis B virus (HBV) is the second most common HCC and the percentage of the total is 10-20 %. Additionally, there exist HCC derived from a superinfection of hepatitis B virus and hepatitis C virus as well as HCC derived from non-B non-C hepatitis and each of them makes up several percent of the total. The common cause of HCC is hepatitis C in Japan and the West and is hepatitis B in other Asian countries and Africa. From 1990, HCC has been the second common cancer which causes death in China. HCC often occurs in a patient with chronic hepatitis or hepatic cirrhosis and it is difficult to find HCC because its symptoms and signs are similar to those of hepatic cirrhosis in progress.

A HCC marker which is now widely used is a carcinoembryonic antigen, AFP (alpha-phetoprotein). Given that an AFP level of 20 mg/ml or more is useful for diagnosing HCC, 60-80 % of HCC cases would be detected. However, when a tumor is small, a sensitivity to the marker is very low, that is, merely 40% of the cases is detectable (see non-patent document 1 described below). Another problem which is associated with use of AFP as a HCC marker is a lack of specificity. For example, significant increase of AFP (20-200 ng/ml) is observed in a considerable number of chronic hepatitis patients (see non-patent document 2 described below). In addition, it is reported that 15-58 % of chronic hepatitis patients and 11-47 % of hepatic cirrhosis patients have a high serum AFP (see non-patent document 3 described below). Accordingly, it is not uncommon that the AFP level overlaps between a HCC patient and a hepatic cirrhosis patient, and therefore, this causes confusion on analysis of a result of an AFP assay. As a result, usability of AFP measurement as a survey tool for patients in danger of HCC has been called in question (see non-patent document 4 described below).
So, it has been propounded that the only circumstance where AFP measurement is judged as reasonable is in use for confirming an initial diagnosis based on an imaging technology (see non-patent document 4 described below). In the past years, although some new potential markers for HCC has been investigated, no marker which is more excellent than AFP has been discovered (see non-patent document 5 described below).

Despite a type of technique, for example, ultrasonography, CT scanning or MRI, a diagnosis by an imaging technique is relatively inaccurate (see non-patent documents 6 and 7 described below). In particular, two lesions which have a possibility to be radiologically similar with HCC are micronodular cirrhosis and dysplastic micronodule. A sensitivity or a specificity of a liver biopsy of microlesion is also insufficient (see non-patent document 8 described below). It may be difficult to identify a sufficiently differentiated cancer from a benign lesion by the biopsy because a limited amount of a sample is normally available by this technique even if the biopsy is a needle biopsy (see non-patent document 9 described below). Accordingly, an adequate molecular marker for identify HCC from a benign liver lesion in a difficult case is still required in spite of advance of an imaging technique.
Also, although a cancer can be detected in the initial stage due to recent advance of ultrasonography, a development of a new serum marker is required because it is often the case that such a well-differentiated cancer is AFP-negative.

As described above, although serum AFP is used for diagnosis as a tumor marker which has a high specificity for HCC and yolk sac tumor, it is difficult to identify a benign disease from HCC by using only a serum AFP level as an index because it become a high level on a benign hepatic disease such as chronic hepatitis and hepatic cirrhosis. Therefore, a diagnostic method using a cancerous change of a sugar chain structure of AFP for identifying HCC from a benign hepatic disease has recently been developed. The method is characterized by measuring AFP-L3 fraction ratio based on a lentils lectin-A (LCA-A) reactivity where lectin affinity electrophoresis is combined with antibody affinity transcription. Although it is suggested that performing a imaging diagnosis such as ultrasonography or X-ray CT, which has been done in the past as a means of diagnosis of HCC, in combination with a measurement of AFP-L3 fraction ratio is useful for identification between HCC and a benign hepatic disease, early diagnosis of HCC as well as prognosis control, development of a new serum marker is now still required because there are a great variety of diagnostic components and a sensitivity and a specificity of a diagnosis by AFP-L3 fraction ratio are still insufficient.

[Nonpatent document 1] Trevisani et al., J. Hepatol., 34:570-575 (2001)
[Nonpatent document 2] Collier et al., Viral Hepatitis Reviews, 4:31-41(1998)
[Nonpatent document 3] Taketa, Hepatology, 12:1420-1432 (1990)
[Nonpatent document 4] Sherman, J. Hepatol., 34:603-605 (2001)
[Nonpatent document 5] Scow et al., Proteomics, 1:1249-1263 (2001)
[Nonpatent document 6] Fong et al., Cancer of the Liver and Binary Tree in Cancer: Principles & Practice Oncology, the 6th ed., 1162-1199 (2001)
[Nonpatent document 7] Murakami et al., Detectability of hypervascular hepatocellular carcinoma by arterial phase images of MR and spiral CT, Acta Radiol., 36:372-376 (1995)
[Nonpatent document 8] Levy et al., Ann. Surg. 234:206-209(2001)
[Nonpatent document 9] Fong et al., Cancer of the Liver and Binary Tree in Cancer: Principles & Practice Oncology, the 6th ed., 1162-1199 (2001)

### DISCLOSURE OF INVENTION

### PROBLEM TO BE SOLVED

The above diagnosis by a serum AFP level has low specificity for hepatic cancer because an increase of the AFP level is also caused by chronic hepatitis, hepatic cirrhosis or pregnancy, and therefore, it has high likelihood of detecting false-positive. Accordingly, development of a serum marker having more excellent sensitivity and higher specificity, which permits early diagnosis and prognostic prediction, has been required.

### MEANS FOR SOLVING PROBLEM

The present inventors performed a large-scale analysis of a serum of patient having an individual disease condition by applying a mass spectrometric technique such as MALDI-TOF MS to a serum sugar chain which was prepared by an existing high-speed exhaustive sugar chain enrichment technique [Nishimura, S, K Niikura, M Kurogochi, T Matsushita, M Fumoto, H Hinou, R Kamitani, H Nakagawa, K Deguchi, N Miura, K Monde, and H Kondo, "High-Throughput Protein Glycomics: Combined Use of Chemoselective Glycoblotting and Maldi-Tof/Tof Mass Spectrometry." Angew Chem Int Ed Engl 44, no. 1 (2004): 91-96]. Then, they established a diagnostic method with a high sensitivity and specificity by correlating the serum sugar chain of a patient to the individual disease condition based on an expression profile of the sugar chain, finding a sugar chain showing a change, and then identifying a serum sugar chain which become a new biomarker, a glycoprotein having the varying sugar chain level, or a group of molecules involved in a biosynthesis pathway of the sugar chain showing a change.
This invention relates to (1) a method for diagnosing a hepatic disease using an expression level of one or more sugar chains as an index, wherein the sugar chains are selected from a group consisting of G2, G8, G9, G10, G11, G12, G14, G15, G16, G17, G18, G20, G21, G23, G26, G27, G30, G31, G33, G37, G45 and G56. In particular, this invention relates to the following methods or uses as described in (2) to (24).
(2) A method for diagnosing HCC using an expression level of one or more sugar chains as an index, wherein the sugar chains are selected from a group consisting of G2, G9, G12, G15, G17, G21, G27, G30, G37, G45 and G56.
(3) The method according to (2), wherein the sugar chains are G37 and/or G56.
(4) A method for predicting prognosis of HCC using an expression level of one or more sugar chains as an index, wherein the sugar chains are selected from a group consisting of G2, G20, G45 and G56.
(5) A method for diagnosing HCC in early stage using an expression level of the sugar chains G20 and/or G26 as an index.
(6) A method for diagnosing hepatopathy using an expression level of one or more sugar chains as an index, wherein the sugar chains are selected from a group consisting of G8, G10, G11, G14, G16, G18, G23, G31 and G33.
(7) A method for diagnosing a benign hepatic disease using an expression level of one or more sugar chains as an index, wherein the sugar chains are selected from a group consisting of G8, G10, G11, G14, G16, G18, G23, G31 and G33.
(8) The method according to any of (1) to (7), wherein the index is a serum expression level of the sugar chains.
(9) A method for diagnosing a hepatic disease characterized by analyzing sugar chains in a biological sample and judging by using an expression level of one or more sugar chains as an index, wherein the sugar chains are selected from a group consisting of G2, G8, G9, G10, G11, G12, G14, G15, G16, G17, G18, G20, G21, G23, G26, G27, G30, G31, G33, G37, G45 and G56.
(10) A method for diagnosing HCC characterized by analyzing sugar chains in a biological sample and judging by using an expression level of one or more sugar chains as an index, wherein the sugar chains are selected from a group consisting of G2, G9, G12, G15, G17, G21, G27, G30, G37, G45 and G56.
(11) The method according to (10), wherein the sugar chains are G37 and/or G56.
(12) A method for predicting prognosis of HCC characterized by analyzing sugar chains in a biological sample and judging by using an expression level of one or more sugar chains as an index, wherein the sugar chains are selected from a group consisting of G2, G20, G45 and G56.
(13) A method for diagnosing HCC in early stage characterized by analyzing sugar chains in a biological sample and judging by using an expression level of the sugar chains G20 and/or G26 as an index.
(14) A method for diagnosing hepatopathy characterized by analyzing sugar chains in a biological sample and judging by using an expression level of one or more sugar chains as an index, wherein the sugar chains are selected from a group consisting of G8, G10, G11, G14, G16, G18, G23, G31 and G33.
(15) A method for diagnosing a benign hepatic disease characterized by analyzing sugar chains in a biological sample and judging by using an expression level of one or more sugar chains as an index, wherein the sugar chains are selected from a group consisting of G8, G10, G11, G14, G16, G18, G23, G31 and G33..
(16) The method according to any one of (9) to (15), wherein the biological sample is a blood.
(17) The method according to any one of (9) to (15), wherein the biological sample is a serum.
(18) Use of one or more sugar chains as a marker for a hepatic disease, wherein the sugar chains are selected from a group consisting of G2, G8, G9, G10, G11, G12, G14, G15, G16, G17, G18, G20, G21, G23, G26, G27, G30, G31, G33, G37, G45 and G56.
(19) Use of one or more sugar chains as a marker for HCC, wherein the sugar chains are selected from a group consisting of G2, G9, G12, G15, G17, G21, G27, G30, G37, G45 and G56.
(20) Use according to (19), wherein the sugar chains are G37 and/or G56..
(21) Use of one or more sugar chains as a marker for predicting prognosis of HCC, wherein the sugar chains are selected from a group consisting of G2, G20, G45 and G56.
(22) Use of the sugar chains G20 and/or G26 as a marker for diagnosing HCC in early stage.
(23) Use of one or more sugar chains as a marker for hepatopathy, wherein the sugar chains are selected from a group consisting of G8, G10, G11, G14, G16, G18, G23, G31 and G33.
(24) Use of one or more sugar chains as a marker for a benign hepatic disease, wherein the sugar chains are selected from a group consisting of G8, G10, G11, G14, G16, G18, G23, G31 and G33.
(25) A method for diagnosing a hepatic disease comprising the following steps:
   (i) analyzing sugar chains in a biological sample; and
   (ii) comparing an expression level of one or more sugar chains of a sample from a healthy individual with those of a test sample, wherein the sugar chains are selected from a group consisting of G2, G8, G9, G10, G11, G12, G14, G15, G16, G17, G18, G20, G21, G23, G26, G27, G30, G31, G33, G37, G45 and G56.
(26) A method for diagnosing HCC comprising the following steps:
   (i) analyzing sugar chains in a biological sample; and
   (ii) comparing an expression level of one or more sugar chains of a sample from a healthy individual with those of a test sample, wherein the sugar chains are selected from a group consisting of G2, G9, G12, G15, G17, G21, G27, G30, G37, G45 and G56.
(27) The method according to (26), wherein the sugar chains are G37 and/or G56.
(28) A method for predicting prognosis of HCC comprising the following steps:
   (i) analyzing sugar chains in a biological sample; and
   (ii) comparing an expression level of one or more sugar chains of a sample from a healthy individual with those of a test sample, wherein the sugar chains are selected from a group consisting of G2, G20, G45 and G56.
(29) A method for diagnosing HCC in early stage comprising the following steps:
   (i) analyzing sugar chains in a biological sample; and
   (ii) comparing an expression level of the sugar chains G20 and/or G26 of a sample from a healthy individual with those of a test sample.
(30) A method for diagnosing hepatopathy comprising the following steps:
   (i) analyzing sugar chains in a biological sample; and
   (ii) comparing an expression level of one or more sugar chains of a sample from a healthy individual with those of a test sample, wherein the sugar chains are selected from a group consisting of G8, G10, G11, G14, G16, G18, G23, G31 and G33.
(31) A method for diagnosing a benign hepatic disease comprising the following steps:
   (i) analyzing sugar chains in a biological sample; and
   (ii) comparing an expression level of one or more sugar chains of a sample from a healthy individual with those of a test sample, wherein the sugar chains are selected from a group consisting of G8, G10, G11, G14, G16, G18, G23, G31 and G33.
(32) The method according to any of (25) to (31), wherein the index is a serum expression level of the sugar chains..

### EFFECT OF INVENTION

According to the present invention, a cancer bearing condition of HCC in a hepatic disease patient can be identified from a benign hepatic disease. An exceptional advantage of a method of the present invention is that the type of a sugar chain as an item for a diagnosis can be arbitrarily chosen from the analytic result which is obtained by one measurement. That is, a burden on the subject is little because there is no need for performing multiple tests and combining the results of them.

### BEST MODE FOR CARRYING OUT THE INVENTION

According to the present invention, all of N-type sugar chains in a blood are recovered from a serum of the subject by Glycoblotting and the quantitative profiles of them are obtained by MALDI-TOF MS. With addition of a known amount of an oligosaccharide as an internal standard, an amount of the sugar chains other than the internal standard in the serum can be easily calculated based on the area of the internal standard on the spectrum. For example, the detectable oligosaccharides are shown in the following Table 1.

**[Table 1]**

| Sugar Chain | Composition of Sugar Chain | m/z |
|---|---|---|
| G1 | (Man)3(GlcNAc)2 | 1340.55 |
| G2 | (Hex)2 + (Man)3(GlcNAc)2 | 1664.65 |
| G3 | (HexNAc)1 (Deoxyhexose)1 + (Man)3(GlcNAc)2 | 1689.69 |
| G4 | (Hex)1 (HexNAc)1 + (Man)3(GlcNAc)2 | 1705.68 |
| G5 | (HexNAc)2 + (Man)3(GlcNAc)2 | 1746.71 |
| G6 | (Hex)3 + (Man)3(GlcNAc)2 | 1826.71 |
| G7 | (Hex)1 (HexNAc)1 (Deoxyhexose)1 + (Man)3(GlcNAc)2 | 1851.74 |
| G8 | (HexNAc)2 (Deoxyhexose)1 + (Man)3(GlcNAc)2 | 1892.77 |
| G9 | (Hex)1 (HexNAc)2 + (Man)3(GlcNAc)2 | 1908.76 |
| G10 | (HexNAc)3 + (Man)3(GlcNAc)2 | 1949.79 |
| G11 | (Hex)4 + (Man)3(GlcNAc)2 | 1988.76 |
| G12 | (Hex)1 (HexNAc)1 (NeuAc)1 + (Man)3(GlcNAc)2 | 2010.79 |
| G13 | (Hex)2 (HexNAc)1 (Deoxyhexose)1 + (Man)3(GlcNAc)2 | 2013.79 |
| G14 | (Hex)1 (HexNAc)2 (Deoxyhexose)1 + (Man)3(GlcNAc)2 | 2054.82 |
| G15 | (Hex)2 (HexNAc)2 + (Man)3(GlcNAc)2 | 2070.81 |
| G16 | (HexNAc)3 (Deoxyhexose)1 + (Man)3(GlcNAc)2 | 2095.84 |
| G17 | (Hex)1 (HexNAc)3 + (Man)3(GlcNAc)2 | 2111.84 |
| G18 | (Hex)5 + (Man)3(GlcNAc)2 | 2150.81 |
| G19 | (Hex)1 (HexNAc)1 (Deoxyhexose)1 (NeuAc)1 + (Man)3(GlcNAc)2 | 2156.85 |
| G20 | (Hex)2 (HexNAc)1 (NeuAc)1 + (Man)3(GlcNAc)2 | 2172.84 |
| G21 | (Hex)1 (HexNAc)2 (NeuAc)1 + (Man)3(GlcNAc)2 | 2213.87 |
| G22 | (Hex)2 (HexNAc)2 (Deoxyhexose)1 + (Man)3(GlcNAc)2 | 2216.87 |
| G23 | (Hex)1 (HexNAc)3 (Deoxyhexose)1 + (Man)3(GlcNAc)2 | 2257.90 |
| G24 | (Hex)2 (HexNAc)3 + (Man)3(GlcNAc)2 | 2273.89 |
| G25 | (Hex)6 + (Man)3(GlcNAc)2 | 2312.87 |
| G26 | (Hex)3 (HexNAc)1 (NeuAc)1 + (Man)3(GlcNAc)2 | 2334.90 |
| G27 | (Hex)1 (HexNAc)2 (Deoxyhexose)1 (NeuAc)1 + (Man)3(GlcNAc)2 | 2359.93 |
| G28 | (Hex)2 (HexNAc)2 (Deoxyhexose)2 + (Man)3(GlcNAc)2 | 2362.93 |
| G29 | (Hex)2 (HexNAc)2 (NeuAc)1 + (Man)3(GlcNAc)2 | 2375.92 |
| G30 | (Hex)1 (HexNAc)3 (NeuAc)1 + (Man)3(GlcNAc)2 | 2416.95 |
| G31 | (Hex)2 (HexNAc)3 (Deoxyhexose)1 + (Man)3(GlcNAc)2 | 2419.95 |
| G32 | (Hex)2 (HexNAc)2 (Deoxyhexose)1 (NeuAc)1 + (Man)3(GlcNAc)2 | 2521.98 |
| G33 | (Hex)1 (HexNAc)3 (Deoxyhexose)1 (NeuAc)1 + (Man)3(GlcNAc)2 | 2563.01 |
| G34 | (Hex)2 (HexNAc)3 (Deoxyhexose)2 + (Man)3(GlcNAc)2 | 2566.01 |
| G35 | (Hex)2 (HexNAc)3 (NeuAc)1 + (Man)3(GlcNAc)2 | 2579.00 |
| G36 | (Hex)3 (HexNAc)3 (Deoxyhexose)1 + (Man)3(GlcNAc)2 | 2582.00 |
| G37 | (Hex)3 (HexNAc)4 + (Man)3(GlcNAc)2 | 2639.02 |
| G38 | (Hex)2 (HexNAc)2 (NeuAcAmide)2 + (Man)3(GlcNAc)2 | 2651.04 |
| G39 | (Hex)2 (HexNAc)2 (NeuAc)2 + (Man)3(GlcNAc)2 | 2681.03 |
| G40 | (Hex)1 (HexNAc)3 (Deoxyhexose)2 (NeuAc)1 + (Man)3(GlcNAc)2 | 2709.07 |
| G41 | (Hex)1 (HexNAc)3 (NeuAc)2 + (Man)3(GlcNAc)2 | 2722.06 |
| G42 | (Hex)2 (HexNAc)3 (Deoxyhexose)1 (NeuAc)1 + (Man)3(GlcNAc)2 | 2725.06 |
| G43 | (Hex)3 (HexNAc)3 (NeuAc)1 + (Man)3(GlcNAc)2 | 2741.06 |
| G44 | (Hex)3 (HexNAc)4 (Deoxyhexose)1 + (Man)3(GlcNAc)2 | 2785.08 |
| G45 | (Hex)2 (HexNAc)2 (Deoxyhexose)1 (NeuAc)2 + (Man)3(GlcNAc)2 | 2827.09 |
| G46 | (Hex)2 (HexNAc)3 (NeuAc)2 + (Man)3(GlcNAc)2 | 2884.11 |
| G47 | (Hex)3 (HexNAc)3 (Deoxyhexose)1 (NeuAc)1 + (Man)3(GlcNAc)2 | 2887.11 |
| G48 | (Hex)2 (HexNAc)3 (Deoxyhexose)1 (NeuAc)2 + (Man)3(GlcNAc)2 | 3030.17 |
| G49 | (Hex)3 (HexNAc)3 (NeuAc)2 + (Man)3(GlcNAc)2 | 3046.17 |
| G50 | (Hex)2 (HexNAc)4 (NeuAc)2 + (Man)3(GlcNAc)2 | 3087.19 |
| G51 | (Hex)4 (HexNAc)4 (NeuAc)1 + (Man)3(GlcNAc)2 | 3106.19 |
| G52 | (Hex)3 (HexNAc)3 (Deoxyhexose)1 (NeuAc)2 + (Man)3(GlcNAc)2 | 3192.23 |
| G53 | (Hex)2 (HexNAc)4 (Deoxyhexose)1 (NeuAc)2 + (Man)3(GlcNAc)2 | 3233.25 |
| G54 | (Hex)3 (HexNAc)3 (NeuAc)3 + (Man)3(GlcNAc)2 | 3351.28 |
| G55 | (Hex)4 (HexNAc)4 (NeuAc)2 + (Man)3(GlcNAc)2 | 3411.30 |
| G56 | (Hex)3 (HexNAc)3 (Deoxyhexose)1 (NeuAc)3 + (Man)3(GlcNAc)2 | 3497.34 |
| G57 | (Hex)4 (HexNAc)4 (NeuAc)3 + (Man)3(GlcNAc)2 | 3716.41 |
| G58 | (Hex)4 (HexNAc)4 (Deoxyhexose)1 (NeuAc)3 + (Man)3(GlcNAc)2 | 3862.47 |
| G59 | (Hex)4 (HexNAc)4 (NeuAc)4 + (Man)3(GlcNAc)2 | 4021.52 |
| G60 | (Hex)4 (HexNAc)4 (Deoxyhexose)1 (NeuAc)4 + (Man)3(GlcNAc)2 | 4167.58 |

[In the Table, "Man" represents mannose, "GlcNAc" represents N-acetylglucosamine, "Hex" represents hexose, and "NeuAc" represents N-acetylneuraminic acid.]
Then, the corresponding hepatic disease condition is deduced from an expression level of one or more sugar chains, or from an increase or decrease of one or more sugar chains, and therefore, a method useful for making diagnosis of a hepatic disease as well as prediction and control of prognosis of the hepatic disease can be obtained.
For example, each of the sugar chains G56, G37, G2, G9, G12, G15, G17, G21, G27, G30 and G45 tends to increase only in the case of HCC. Above all, an expression level of the sugar chain G56 has no dependency on hepatic fibrosis and is unaffected by the presence or absence of hepatitis, and therefore, this sugar chain can be used as a marker for identifying significantly the HCC group (See Example 1 below.). In addition, the diagnostic efficiency can be further improved by combining the variations of expression levels of multiple sugar chains. For example, more precise HCC diagnosis can be performed by using G56 in combination with G37.

A marker which varies in the case of hepatopathy includes each of the sugar chains G8, G10, G11, G14, G16, G18, G23, G31 and G33. The expression level of these sugar chains varies unaffectedly by the presence of a cancer, and therefore, it can be a promising marker useful for diagnosis in the stage of a benign hepatic disease (See Example 2 below.). Above all, it would appear that the G8 sugar chain is a preferable marker for diagnosis in the stage of a benign hepatic disease because this sugar chain shows a great significant difference.

There also exists a sugar chain which is highly expressed with the progression of a hepatic disease. Each of the sugar chains G20 and G26 is highly expressed in a hepatic disease and it is recognized that these sugar chains tend to be more highly expressed in a cancer bearing condition, and therefore, it would appear that these sugar chains are a promising marker for diagnosis in the stage of hepatopathy. Particularly, the G20 sugar chain has a pronounced tendency to such increase of an expression level, and therefore, this sugar chain is highly significant because a benign hepatic disease often progresses to a cancer bearing condition (See Example 3 below.). Additionally, there is a possibility that the G20 and/or G26 sugar chains are useful for diagnosing HCC in early stage by using them in combination with a marker for diagnosis in the stage of a benign hepatic disease such as the G8 sugar chain above.

On the other hand, there exists a sugar chain such as G49 and G54 which shows a reduced expression level in hepatopathy. These sugar chains are also useful for a marker of hepatopathy.
Meanwhile, a higher-expression level of the sugar chains G56, G2, G45 and G20 results in a decrease in the survival rate. Accordingly, these sugar chains are effective as a marker for control of prognosis. It is preferable that an expression level of one or more sugar chains selected from a group consisting of G20, G45 and G56 can be used as a marker for predicting prognosis of HCC (See Example 4 below.). It is more preferable that an expression level of the G56 sugar chain can be used for as a marker for predicting prognosis of HCC.
The present invention is illustrated in more details by the following Examples, but it should not be construed to be limited thereto.

### Example 1

All of N-type sugar chains in a blood were recovered from a serum of a total of 216 of patients with HCC and/or hepatitis B or C as well as healthy individuals by Glycoblotting and the quantitative profiles of them were obtained by MALDI-TOF MS. An amount of the sugar chains in the serum listed in Table 1 was calculated based on the area on the spectrum of a known amount of an oligosaccharide which was added as an internal standard. The breakdown of the serum of patients used herein is shown in Table 2 and 3 below.

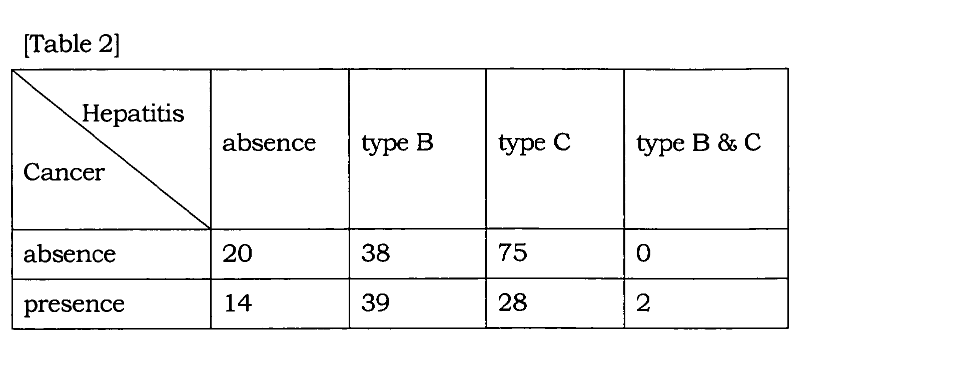

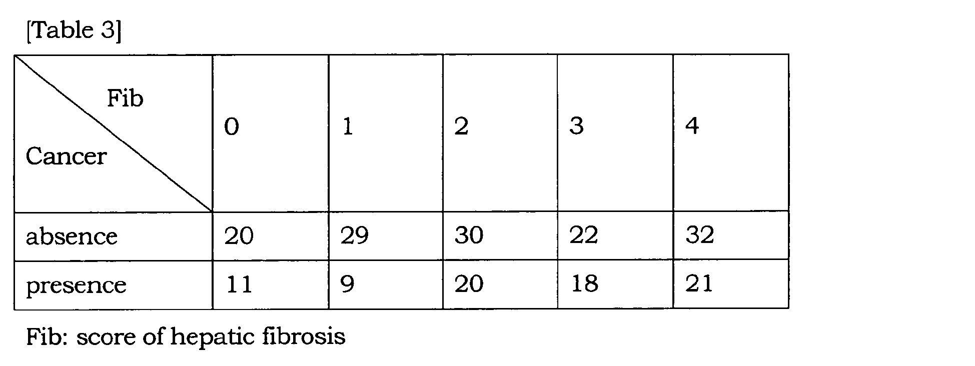

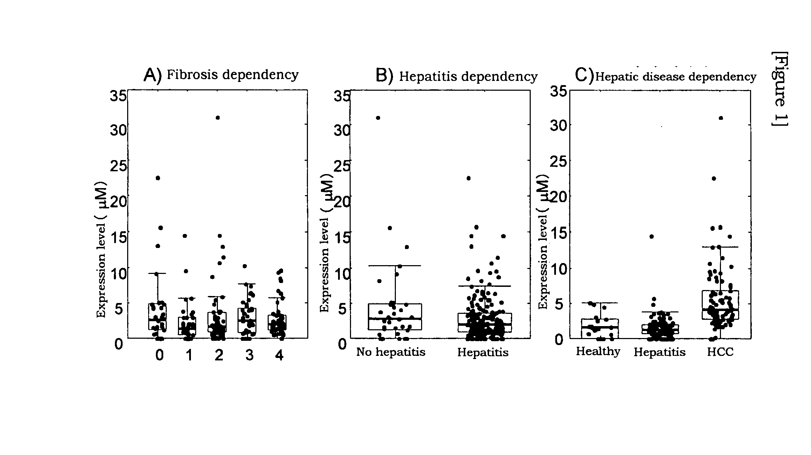

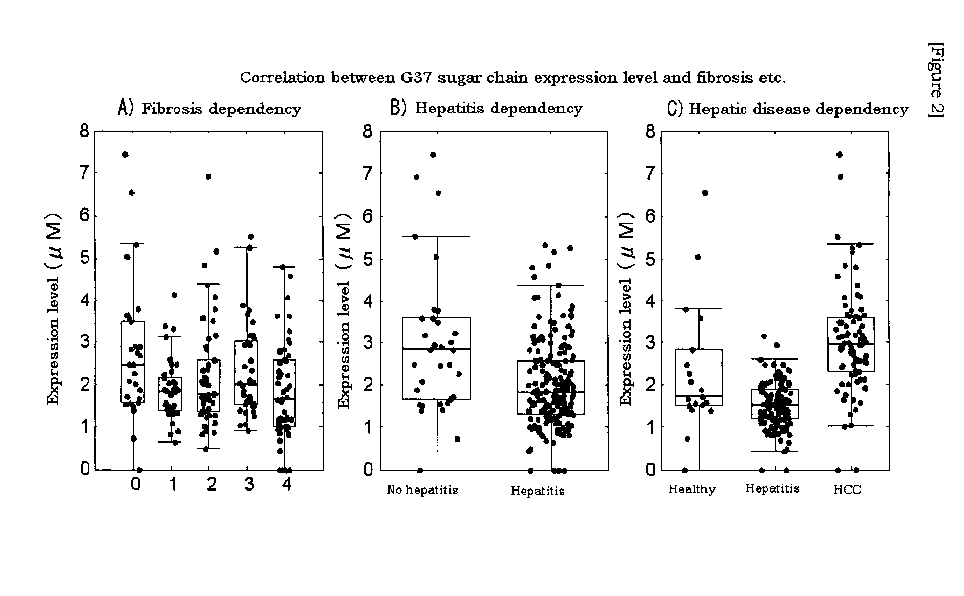

Then, a quantitative value of each sugar chain in each sample was defined as an expression level of the sugar chain and an investigation by statistical procedures about the presence of a sugar chain which is unaffected by hepatitis or hepatic fibrosis and varies only in the case of a hepatic cancer was performed. As a result, it was found that an expression level of the sugar chains G56, G37, G2, G9, G12, G15, G17, G21, G27, G30 and G45 tended to increase only in the case of HCC. A boxplot of the sugar chains G56 and G37 which show a great significant difference is shown in Figure 1 and 2.

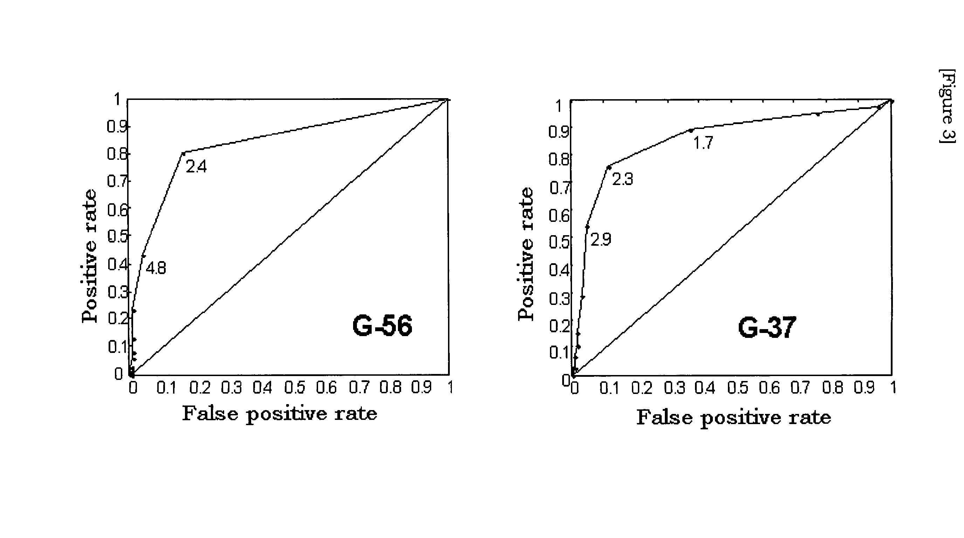

An expression level of the G56 sugar chain has no dependency on hepatic fibrosis and is unaffected by the presence or absence of hepatitis. On the other hand, an expression of the G56 sugar chain increased specifically in a group of patients with a cancer bearing condition of HCC, and therefore, the HCC group was significantly identified from a group of healthy individuals and patients with a benign hepatic disease. A ROC curve of the G56 sugar chain is shown in Figure 3 (left). In the case where a threshold level is set at 2.4 µM, the sensitivity was 80 % and the specificity was 85 %. Accordingly, the possibility that the G56 sugar chain would become an excellent method for diagnosis was suggested. A ROC curve of the G37 sugar chain is shown in Figure 3 (right). In the case where a threshold level is set at 2.3 µM, a result that an effective diagnosis can be performed is demonstrated. Although each of the sugar chains G56 and G37 is independently effective for diagnosis, it would appear that a combination use of these sugar chains can further reduce an error of diagnosis.

### Example 2

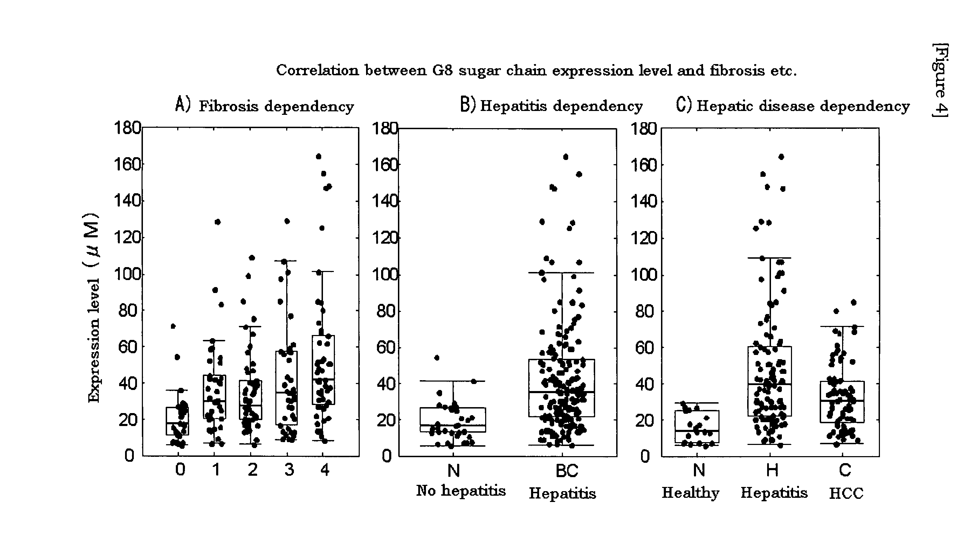

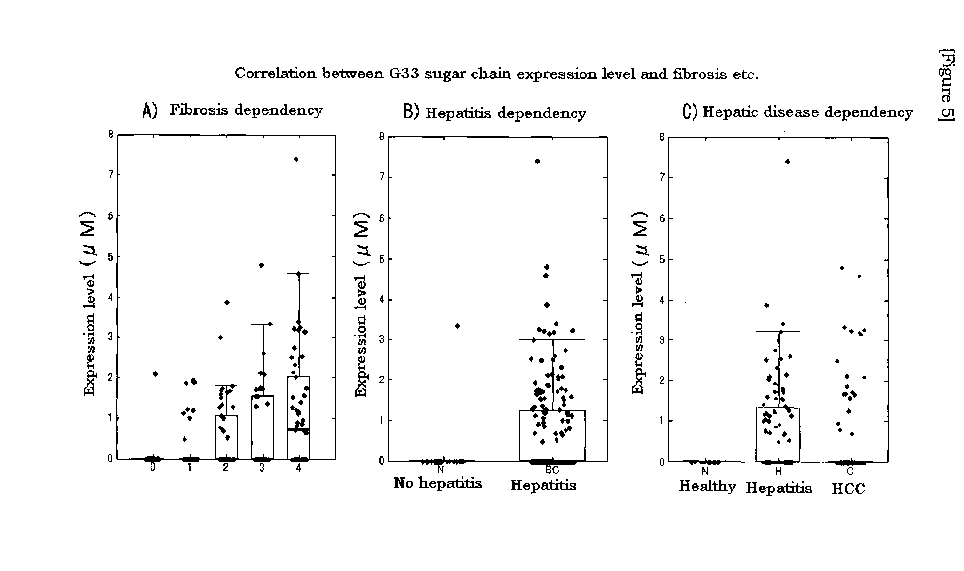

In this example, an investigation of a sugar chain group which is unaffected by the presence of a cancer and varies in the case of hepatopathy was performed. As a result, it was found that each of the sugar chains G8, G10, G11, G14, G16, G18, G23, G31 and G33 had such tendency. A result as to the sugar chains G8 and G33 is shown in Figure 4 and 5, respectively. It would appear that these sugar chains are a promising candidate of a marker for diagnosis in the stage of a benign hepatic disease stage.

### Example 3

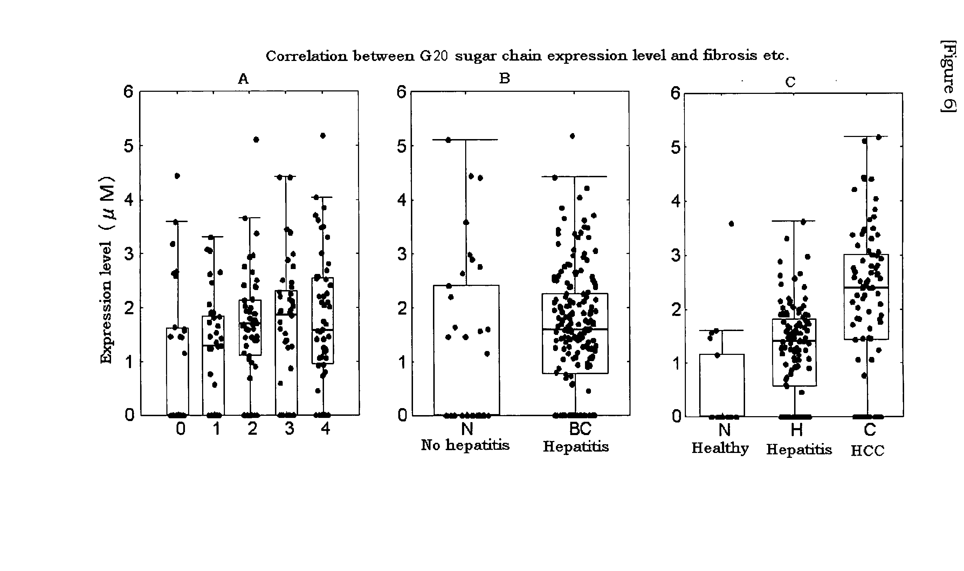

In this example, an identification of a sugar chain which was highly expressed in a hepatic disease and was more highly expressed in a cancer bearing condition was performed. It was found that the sugar chains G20 and G26 tended to be highly expressed with the progression of a hepatic disease. The result of the sugar chain G20 is shown in Figure 6. It would appear that these sugar chains are a promising candidate of a marker for diagnosis in the stage of hepatopathy.

### Example 4

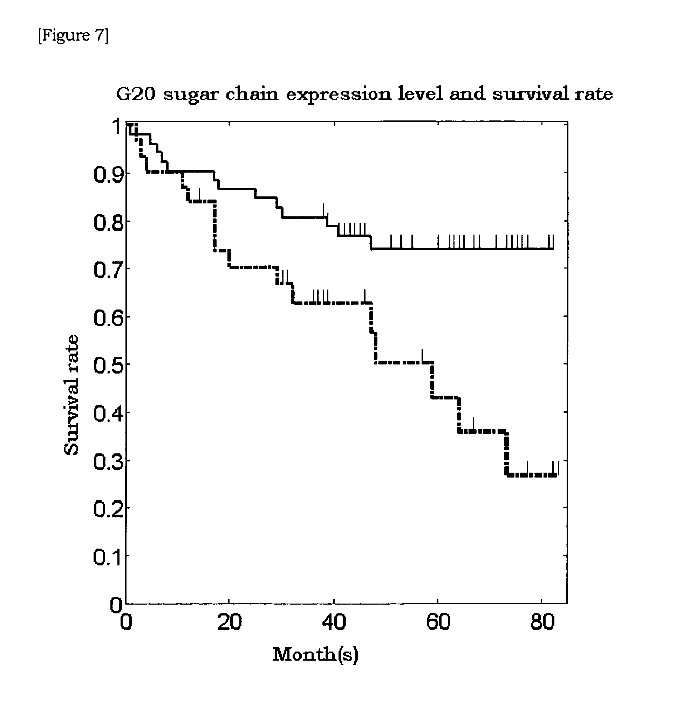

It was found by evaluating a survival rate for each sugar chains that a higher-expression level of the sugar chains G20, G45 and G56 resulted in a worse prognosis. The confirmation results of an efficacy of each sugar chains by using a survival rate and a logrank test are shown in Figures 7-9, respectively. It was found that a higher-expression level of these sugar chains resulted in a worse prognosis. In addition, because it is apparent from the logrank test that this result is not caused by contingency, it would appear that these sugar chains are also useful for prediction of prognosis.

### INDUSTRIAL APPLICABILITY

According to the present invention, a cancer bearing condition of HCC in a hepatic disease patient can be identified from a benign hepatic disease, and therefore, HCC can be significantly identified for diagnosis with or without a benign hepatic disease. In addition, according to the present invention, prediction of prognosis of a patient with a hepatic disease becomes possible. As a result, it would be greatly expected that a method of the present invention can contribute to a therapeutic effect and an improvement of prognosis by offering a proper treatment.

### BRIEF DESCRIPTION OF DRAWINGS

[Figure 1] A correlation between an expression level of the G56 sugar chain and hepatic fibrosis or a hepatic disease is shown. An expression level of the G56 sugar chain is plotted against A) each score of fibrosis; B) a group of patients with or without hepatitis; and C) a group of healthy individuals, hepatitis patients and HCC patients.
[Figure 2] A correlation between an expression level of the G37 sugar chain and hepatic fibrosis or a hepatic disease is shown. An expression level of the G37 sugar chain is plotted against A) each score of fibrosis; B) a group of patients with or without hepatitis; and C) a group of healthy individuals, hepatitis patients and HCC patients.
[Figure 3] Each ROC (Receiver Operation Characteristic) curve of the G56 sugar chain (left) and the G37 sugar chain is shown. The vertical axis is a positive rate and the horizontal axis is a false-positive rate.
[Figure 4] A correlation between an expression level of the G8 sugar chain and hepatic fibrosis or a hepatic disease is shown. An expression level of the G8 sugar chain is plotted against A) each score of fibrosis; B) a group of patients with or without hepatitis; and C) a group of healthy individuals, hepatitis patients and HCC patients.
[Figure 5] A correlation between an expression level of the G33 sugar chain and hepatic fibrosis or a hepatic disease is shown. An expression level of the G33 sugar chain is plotted against A) each score of fibrosis; B) a group of patients with or without hepatitis; and C) a group of healthy individuals, hepatitis patients and HCC patients.
[Figure 6] A correlation between an expression level of the G20 sugar chain and hepatic fibrosis or a hepatic disease is shown. An expression level of the G20 sugar chain is plotted against A) each score of fibrosis; B) a group of patients with or without hepatitis; and C) a group of healthy individuals, hepatitis patients and HCC patients.
[Figure 7] An evaluation of a survival rate base on an expression level of the G20 sugar chain is shown. Each overall survival rate of two groups which was made by dividing the expression level distribution of the G20 sugar chain at 63 percentile from the low side of the expression level is shown. The lower polygonal line indicates a high expression level group (more than 63 percentile). A significant difference was confirmed by a logrank test.

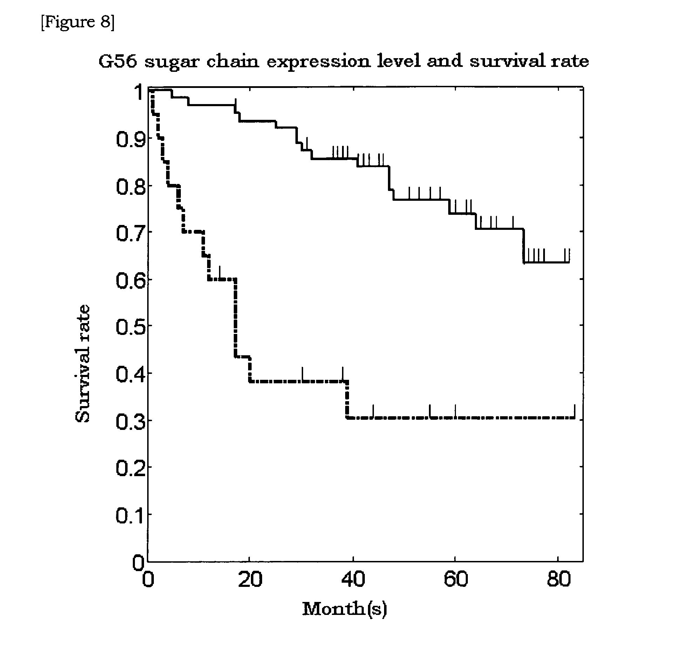

[Figure 8] An evaluation of a survival rate base on an expression level of the G56 sugar chain is shown. Each overall survival rate of two groups which was made by dividing the expression level distribution of the G56 sugar chain at 76 percentile from the low side of the expression level is shown. The lower polygonal line indicates a high expression level group (more than 76 percentile). A significant difference was confirmed by a logrank test.

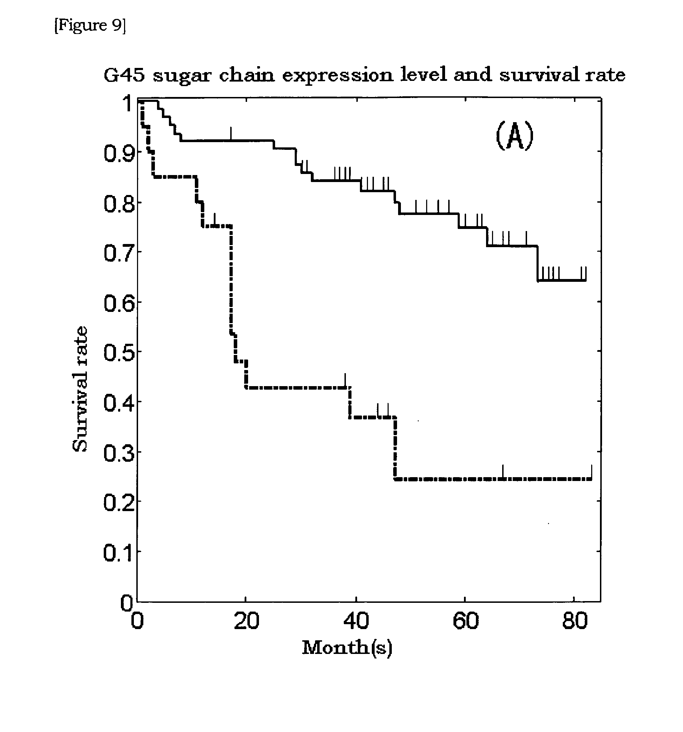

[Figure 9] An evaluation of a survival rate base on an expression level of the G45 sugar chain is shown. Each overall survival rate of two groups which was made by dividing the expression level distribution of the G45 sugar chain at 76 percentile from the low side of the expression level is shown. The lower polygonal line indicates a high expression level group (more than 76 percentile). A significant difference was confirmed by a logrank test.

## Claims

1. A method for diagnosing a hepatic disease using an expression level of one or more sugar chains as an index, wherein the sugar chains are selected from a group consisting of G2, G8, G9, G10, G11, G12, G14, G15, G16, G17, G18, G20, G21, G23, G26, G27, G30, G31, G33, G37, G45 and G56.

2. A method for diagnosing hepatocellular carcinoma using an expression level of one or more sugar chains as an index, wherein the sugar chains are selected from a group consisting of G2, G9, G12, G15, G17, G21, G27, G30, G37, G45 and G56.

3. The method according to claim 2, wherein the sugar chains are G37 and/or G56.

4. A method for predicting prognosis of hepatocellular carcinoma using an expression level of one or more sugar chains as an index, wherein the sugar chains are selected from a group consisting of G2, G20, G45 and G56.

5. A method for diagnosing hepatocellular carcinoma in early stage using an expression level of the sugar chains G20 and/ or G26 as an index.

6. A method for diagnosing hepatopathy using an expression level of one or more sugar chains as an index, wherein the sugar chains are selected from a group consisting of G8, G10, G11, G14, G16, G18, G23, G31 and G33.

7. A method for diagnosing a benign hepatic disease using an expression level of one or more sugar chains as an index, wherein the sugar chains are selected from a group consisting of G8, G10, G11, G14, G16, G18, G23, G31 and G33.

8. The method according to any of claims 1 to 7, wherein the index is a serum expression level of the sugar chains.

9. Use of one or more sugar chains as a marker for a hepatic disease, wherein the sugar chains are selected from a group consisting of G2, G8, G9, G10, G11, G12, G14, G15, G16, G17, G18, G20, G21, G23, G26, G27, G30, G31, G33, G37, G45 and G56.

10. Use of one or more sugar chains as a marker for hepatocellular carcinoma, wherein the sugar chains are selected from a group consisting of G2, G9, G12, G15, G17, G21, G27, G30, G37, G45 and G56.

11. Use according to claim 10, wherein the sugar chains are G37 and/or G56..

12. Use of one or more sugar chains as a marker for predicting prognosis of hepatocellular carcinoma, wherein the sugar chains are selected from a group consisting of G2, G20, G45 and G56.

13. Use of the sugar chains G20 and/or G26 as a marker for diagnosing hepatocellular carcinoma in early stage.

14. Use of one or more sugar chains as a marker for hepatopathy, wherein the sugar chains are selected from a group consisting of G8, G10, G11, G14, G16, G18, G23, G31 and G33.

15. Use of one or more sugar chains as a marker for a benign hepatic disease, wherein the sugar chains are selected from a group consisting of G8, G10, G11, G14, G16, G18, G23, G31 and G33.
